# EUROPÄISCHE PATENTSCHRIFT

(11) **EP 1 534 360 B1**
(45) Veröffentlichungstag und Bekanntmachung des Hinweises auf die Patenterteilung: **14.06.2006**
(21) Anmeldenummer: 04730203.9
(22) Anmeldetag: 29.04.2004
(51) Int. Cl.: A61M 1/36

(54) **TRAGBARE HERZLUNGENMASCHINE**
PORTABLE HEART AND LUNG MACHINE
COEUR-POUMON ARTIFICIEL PORTABLE

(30) Priorität: 09.05.2003 DE 20307256 U
(43) Veröffentlichungstag der Anmeldung: 01.06.2005
(60) Teilanmeldung: 05022470.8
(73) Patentinhaber: LIFEBRIDGE Medizintechnik AG, 84539 Ampfing (DE)
(72) Erfinder: KIRCHHOF, Karsten, 36151 Burghaun (DE)
(74) Vertreter: Manitz, Finsterwald & Partner GbR
(86) Internationale Anmeldenummer: PCT/EP2004/004559
(87) Internationale Veröffentlichungsnummer: WO 2004/098678

(56) Entgegenhaltungen:
- DE-C- 19 905 937
- DE-U- 20 008 961
- US-A- 5 308 320

## Beschreibung

Die Erfindung betrifft eine mobile Herzlungenmaschine zur Aufrechterhaltung des Kreislaufs.

Zum Aufrechterhalten des Kreislaufs eines Menschen zur Vermeidung oder Beseitigung von Kreislaufstillstand oder Minderperfusion während einer Operation, infolge eines Unfalls oder eines sonstigen organischen Versagens wird diesem mittels einer Herzlungenmaschine sauerstoffarmes venöses Blut mit einer Kanüle entnommen und mit einer Blutpumpe einem Oxygenator zugeführt. In dem Oxygenator, der die Funktion einer künstlichen Lunge übernimmt, wird das Blut mit Sauerstoff angereichert und CO₂ entfernt. Anschliessend wird das sauerstoffreiche, arterielle Blut nach Reinigung in einem arteriellen Filter durch eine Kanüle dem Kreislauf des Patienten wieder zugeführt. Derartige Herzlungenmaschinen werden für einen stationären Einsatz in Krankenhäusern verwendet.

Aus der DE 43 43 334 A1 ist eine Herzlungenmaschine bekannt, welche für einen mobilen Einsatz eine Tragkonstruktion aufweist, die vorne und hinten mit Traggriffen versehen, brückenförmig aufgebaut und mit nach unten vorstehenden Standfüßen versehen ist. Für die Fixierung aller Bestandteile der Herzlungenmaschine sind entsprechende Befestigungsmittel vorgesehen. Allerdings sind bei dieser Herzlungenmaschine die funktionswichtigen Elemente frei zugänglich angeordnet und somit nicht vor Beschädigungen geschützt, die auch während des Einsatzes die Funktionsfähigkeit beeinträchtigen können. Darüber hinaus muss die Maschine immer von zwei Personen getragen werden.

Ferner ist aus der DE 197 02 098 A1 eine mobile Herz-Lungen-Maschine mit einem Schlauchset bekannt, welche aus einer Schlinge zur Zuführung von Blut zu einer Arterie, einer zweiten Schlinge zur Abführung von Blut aus einer Vene, einem venösen Reservoir, einem Oxygenator, einer vorzugsweise als Rollenpumpe ausgebildeten Blutpumpe und einem Sauerstoffspender besteht, wobei der Sauerstoffspender ein Sauerstoffkonzentrator ist, der mit der Pumpe antriebsseitig verbunden ist. Darüber hinaus weist die Maschine Regler für den Sauerstoffkonzentrator und die Förderleistung der Pumpe sowie Anschlüsse für eine dezentrale Energieversorgung und/oder für einen Elektroenergiespeicher auf. Obwohl diese Herz-Lungen-Maschine zwar speziell für einen mobilen Einsatz gedacht ist, lässt sie sich jedoch aufgrund ihrer Grösse und ihres Gewichts nur schwer von einer Person handhaben.

Wegen der beengten Platzverhältnisse in einem Notarztwagen ist es so gut wie unmöglich, solche Herzlungenmaschinen bei einem Notfalleinsatz bereits betriebsbereit mitzuführen. Bei der gebotenen Eile bei Notfalleinsätzen, insbesondere bei Patienten, bei denen ein Versagen der Herz-Lungen-Funktion vorliegt, ist es jedoch besonders nachteilig, wenn derartige Herzlungenmaschinen vor dem Einsatz erst fertig montiert werden müssen. Darüber hinaus sind die vorgenannten Herzlungenmaschinen nach dem Gebrauch vor einem erneuten Einsatz in einem aufwendigen Verfahren neu aufzubauen und zu reinigen. Dadurch ist ein sofortiger Wiedereinsatz nicht möglich.

Die DE 199 05 937 offenbart eine mobile Herzlungenmaschine nach dem oberbegriff der Ansprüche 1 und 10, die aus zwei Modulen besteht, wobei in einem Modul die blutführenden Elemente und in dem anderen Modul die Antriebs- und Steuerungselemente untergebracht sind.

Aus der DE-U-200 08 961 ist eine Infusionspumpe mit einem mobilen Basismodul bekannt, das einen Pumpenteil, einen Energiespeicher und eine Einstellvorrichtung aufweist. Ferner ist ein Bedienmodul vorgesehen, welches an das Basismodul zur Energieübertragung und zur Datenübertragung ankoppelbar ist. Das Basismodul besitzt eine minimierte Benutzeroberfläche.

Es ist die Aufgabe der vorliegenden Erfindung, Vorrichtungen zu schaffen, die für einen mobilen Einsatz einer Herz-Lungen-Maschine angepasst sind.

Die Lösung dieser Aufgabe erfolgt durch die Merkmale der unabhängigen Ansprüche.

Die abhängigen Ansprüche definieren bevorzugte Ausführungsformen der Erfindung.

Durch den modularen Aufbau der Herzlungenmaschine wird sichergestellt, dass die Herzlungenmaschine einfach und rasch auseinandergebaut und wieder zusammengebaut werden kann.

Die Herzlungenmaschine wird bevorzugt in 3 Funktionseinheiten (Module) aufgeteilt.

Die Funktionseinheit, in der die blutberührenden Einbauteile, wie Reservoir (4), Pumpenkopf (5), Oxygenator (6) mit Wärmetauscher (7) oder arterieller Filter (8) eingebaut sein können, wird hier als Patientenmodul (1) bezeichnet.

Die Funktionseinheit, in der der Pumpenantrieb (9) mit mechanischer Handkurbel (10), der wesentliche Teil der Sensorik (11) und ein Backup-Akku (12) untergebracht sind, wird Steuermodul (2) genannt.

Die Grundeinheit mit Bedienoberfläche (13) (z.B. Touchscreen), einem Computer (14), z.B. einem Klein-PC, und einer Hauptenergieversorgung (15) wird als Basismodul (3) bezeichnet.

Der Ausdruck "Modul" bedeutet in der vorliegenden Erfindung eine Einheit, in der die jeweiligen Teile stabil an einem Träger befestigt sind. Dieser Träger kann im einfachsten Fall ein Gestell sein. Bevorzugt ist jedoch ein offenes oder geschlossenes Gehäuse. Die einzelnen Teile der Einheit sind zwar stabil mit dem Träger und/oder stabil miteinander verbunden, die Verbindung ist jedoch lösbar. Bei den Verbindungen handelt es sich bevorzugt um Steckverbindungen. Bevorzugt befinden sich alle Funktionsteile in einem Gehäuse, so dass sie vor mechanischen Beschädigungen geschützt sind. Weniger empfindliche Teile können auch außen am Gehäuse angebracht sein. Der Aufbau der Module ist nur insoweit eingeschränkt, als die einzelnen Module für sich oder in Kombination von zwei oder drei Modulen die Eigenschaft aufweisen müssen, von einer Person getragen werden zu können. Hierfür sollten die Module kompakt sein und dürfen nicht sperrig aufgebaut sein. Um von einer Person getragen werden zu können, entspricht die Größe der Herzlungenmaschine bevorzugt etwa der eines großen Aktenkoffers, und das Gewicht übersteigt vorzugsweise 20 kg nicht.

Die erfindungsgemäße Herzlungenmaschine hat die Aufgabe, den Herz-Lungen-Kreislauf aufrecht zu erhalten und/oder diesen zu ersetzten. Eine solche Herzlungenmaschine besteht üblicherweise aus einer venösen Zuführung des Patientenblutes, einem Reservoir zum Volumenausgleich, einer Blutpumpe zur aktiven Unterstützung des Blutkreislaufs, einem Oxygenator zur Sauerstoffanreicherung und CO₂-Abführung, einem Wärmetauscher zur Temperierung (Erwärmen oder Abkühlen) der Bluttemperatur, einem arteriellen Filter mit Bypass zur Filtrierung des Blutes, Ventilen zur Schaltung eines internen Kreislaufs, sowie einer arteriellen Linie, um dem Patienten das Blut erneut zuzuführen. Der interne Bypass wird benötigt, µm das Gerät vor Anschluss an den Patientenkreislauf mit einem Blutersatzstoff zu füllen und das Gesamtsystem zu entlüften (Priming). Die internen Volumina sind dabei äußerst klein zu halten, um so das Volumen des Blutersatzstoffes möglichst gering halten zu können. Um dieses Merkmal zu erfüllen, besteht auch die Möglichkeit, die Blutführungswege im Gehäuse zu integrieren bzw. mit in das Kunststoffgehäuse einzugießen.

Um die sehr sensible Sensorik (Messfühler) in bzw. an den entsprechenden Messstellen ein- und anzubauen, wird das Steuermodul zusammen mit dem Patientenmodul vorzugsweise vormontiert und getestet, so dass der potentielle Anwender eine funktionsfähige Baueinheit in Kassettenform bekommt, die nur noch auf das Basismodul aufgesetzt und arretiert werden muß. Die Kassette mit den blutberührenden Teilen, wie venöse Zuführung des Patientenblutes, Reservoir, Blutpumpe, Oxygenator, arterieller Filter mit Bypass und Ventilen, wird nach der Anwendung vom Steuermodul getrennt und entsorgt. Das Steuermodul selbst wird zur Wiederaufarbeitung an den Hersteller zurückgesandt. Die im Steuermodul und im Basismodul abgelegten Patientendaten können über eine entsprechende Datenleitung/Schnittstelle ausgelesen werden und der behandelnden Klinik für die Patientenakte oder für statistische Zwecke zur Verfügung gestellt werden.

Durch die oben beschriebene Vorgehensweise wird sichergestellt, dass der Aufbau fehlerfrei durchgeführt wird. Die Fehlerquelle "Aufbau vor Ort" unter erschwerten Bedingungen wird somit im wesentlichen auf das Befüllen und Entlüften der Herzlungenmaschine begrenzt.

Die notwendige Software zur Steuerung und Regelung aller Prozesse und Vorgänge wird bevorzugt von einem Klein-PC im Basismodul übernommen. In einem Grundprogramm werden die Basisbetriebsdaten vorgegeben. Möglicherweise notwendige Veränderungen können über das Eingabeinstrument (z.B. Touchscreen) vorgenommen werden. Auch diese Änderungen können gespeichert werden.

Zur Energieversorgung der tragbaren Herzlungenmaschine stehen zwei Akkus zur Verfügung. Im Basismodul wird über den Haupt-Akku die Grundversorgung sichergestellt. Im Steuermodul ist ein Backup-Akku vorgesehen, der über einen begrenzten Zeitraum einen Notbetrieb aufrecht erhält.

Ein weiterer großer Vorteil der Erfindung besteht darin, dass das Steuermodul zusammen mit dem Patientenmodul nach Grundeinstellung über das Basismodul auch autark, das heißt ohne Basismodul, mit einer externen Spannungsversorgung z.B. über eine Autobatterie, betrieben werden kann. Es können zwar dann keine Änderungen oder Anpassungen der Regelungsparameter vorgenommen werden, jedoch ist man dann mit der Anzahl der Anwendungen nicht mehr auf die Anzahl der Basismodule angewiesen.

In Abgrenzung zu anderen bekannten Veröffentlichungen wie z.B. die DE 199 05 937 Cl, handelt es sich bei der vorliegenden Erfindung um eine Herzlungenmaschine, die leicht von einer Person tragbar ist und aus verschiedenen Kassetten besteht, in der die blutführenden Teile, die Steuerungselemente und Sensoren zur Messung z. B. der Bluttemperatur, des Blutdrucks, der Sauerstoffsättigung, des Volumenstroms, der internen Drücke, sowie die Basisversorgung mit Energie und Software untergebracht sind.

Mit entsprechenden Fühlern und Sensoren werden unter anderem die Temperaturen am arteriellen Eingang, am arteriellen Ausgang sowie am Oxygenator, der Druck am venösen Eingang, am arteriellen Ausgang, zwischen Pumpe und Oxygenator , der Druck der O₂-Zufuhr, die Sauerstoffsättigung am venösen Eingang und arteriellen Ausgang, die Luftblasenerkennung am venösen Eingang und arteriellen Ausgang, sowie der Blutvolumenstrom am arteriellen Ausgang gemessen.

Die einzelnen Module können im Herstellerwerk montiert und in Gehäusen untergebracht werden, die alle Einbauteile vor mechanischer Beschädigung von außen schützen.

Die Blutführung im Inneren des Patientenmoduls wird nicht, wie aus der DE 197 02 098 A1 bekannt, durch ein vorkonfektioniertes Schlauchset vorgenommen, sondern durch ein vorgeformtes Blutführungssystem. Der Vorteil dieser Art der Blutführung liegt in der geringeren mechanischen Belastung des Blutes, der besseren Hämodynamik sowie darin, dass geringere Scherkräfte auftreten.

Die durch eine Person tragbare und bedienbare Herzlungenmaschine kann sowohl direkt vor Ort bei einem Notfalleinsatz als auch in einer Klinik oder einer kardiologischen Praxis eingesetzt werden. Es ist auch der reine stationäre Einsatz in Kliniken denkbar.

Die Verbindung zum Steuermodul erfolgt vorzugsweise funktional, sowohl elektrisch als auch mechanisch. Die elektrischen Verbindungen können die gesamte Sensorik dem Steuermodul zur Auswertung bereitstellen und die Ansteuerung der Ventile ermöglichen. Die mechanische Verbindung ermöglicht die Kraftübertragung vom Pumpenantrieb im Steuermodul zum Pumpenkopf im Patientenmodul.

Bei Ausfall des Steuermodul kann dem Anwender durch ein Handrad (Kurbel) die Möglichkeit gegeben werden, die wesentliche Funktion der Herzlungenmaschine durch manuelle Betätigung aufrechtzuerhalten.

Das Steuermodul besteht vorzugsweise aus einem Gehäuse mit den Baugruppen der gesamten Mess-, Steuer- und Regelelektronik, dem Pumpenantrieb, den Ventilen für die Einstellung des internen Bypasses, der Übergangsenergieversorgung, den Bedieneinheiten für den Notbetrieb, den Anschlüssen für die externe Sensorik (Temperatur, Druck und Sauerstoffsättigung, Volumenstrommessung), den Verbindungen zum Patientenmodul, den Verbindung zum Basismodul, dem Speichermedium zur Übergabe von Informationen von einem Basismodul zu einem anderen Basismodul.

Das Steuermodul kann sich baulich innerhalb des Patientenmoduls befinden und wird vor Auslieferung werksseitig mit diesem verbunden. Die Funktionsprüfung und anschließende Kalibrierung erfolgen gemeinsam. Beide Module werden dem Anwender als Einheit zur Verfügung gestellt. Nach Gebrauch können Patientenmodul und Steuermodul vom Anwender voneinander getrennt werden. Das Patientenmodul mit seinen blutführenden Teilen kann vom Anwender entsprechend entsorgt werden. Das Steuermodul kann anschließend an den Hersteller zurückgesandt werden, um nach angemessenen Überprüfungen erneut eingesetzt zu werden.

Das Basismodul besteht bevorzugt aus einem Gehäuse, das einen Klein-PC, ein LCD-Display mit Bedienoberfläche z. B. einen Touchscreen-Bildschirm, die Hauptenergieversorgung, die Ladeeinrichtungen sowie die Verbindung zum Steuermodul enthält.

Die Aufgaben des Basismoduls sind die Bereitstellung der notwendigen Energie, die Steuerung und Überwachung des Ladevorgangs, die fehlertolerante und übersichtliche Bedienung des Gesamtsystems, die Aufzeichnung aller relevanten Betriebsdaten, die Dokumentation der vom Anwender durchgeführten Aktionen und die Verbindung zu externen DV Einrichtungen zur Übergabe der aufgezeichneten Informationen.

Die elektrische Verbindung vom Basismodul zum Steuermodul kann z. B. vierpolig ausgeführt sein und dient der Energie- und Informationsübertragung.

Das Basismodul kann stets beim Anwender verbleiben und von ihm vor dem Einsatz mit der Einheit aus Steuermodul und Patientenmodul verbunden werden.

Der größte Vorteil gegenüber bereits bekannten Herzlungenmaschinen ist der, dass das Steuermodul immer wieder zum Hersteller zurückkommen kann und so eine sehr häufige Kontrolle der steuerungsrelevanten Bauteile stattfinden kann, so dass sich Softwareupdates sehr einfach auf alle Anwendungen übertragen lassen und sich die sehr sensible Sensorik immer wieder neu bei der Werksmontage überprüfen, einstellen auf Funktion prüfen und kalibrieren lässt.

Der komplizierteste Vorgang beim Zusammenbau der einzelnen Systemkomponenten ist die Verbindung vom Patientenmodul mit dem Steuermodul. Dieser Vorgang kann nun aber werksseitig durchgeführt werden und entlastet somit den Anwender. Vor allem im Notfalleinsatz werden hiermit auch mögliche Fehlerquellen ausgeschlossen.

Wie erwähnt können Patientenmodul und Steuermodul vor Verlassen des Werks gemeinsam einer Funktionsprüfung und Kalibrierung unterzogen werden. Damit wird höchste Genauigkeit von gemessenen Sensordaten erzielt und eine Fehlfunktion durch unsachgemäßes Zusammenführen der vielpoligen elektrischen und mechanischen Verbindung zwischen Patientenmodul und Steuermodul durch den Anwender ausgeschlossen.

Da die komplizierteste und fehlerträchtigste Verbindung nun im Werk durchgeführt werden kann, kann ihre technische Auslegung auch einfacher erfolgen und z.B. auch mit Spezialvorrichtungen ausgeführt werden. Dies kann in der Entwicklung und späteren Fertigung zu relevanten Einsparungen führen.

Da die Verbindung vom Basismodul zum Steuermodul lediglich zur Informations- und Energieübertragung notwendig ist, ist es möglich, auch nachfolgende Generationen der erfindungsgemäßen Herzlungenmaschine, evtl. nach einem Software-Update des Basismoduls, mit dem vorhandenen Basismodul der ersten Generation einzusetzen. Damit sichert der Anwender seine Investition in das Basismodul auch für die Zukunft.

Dieser Vorteil soll am Beispiel von möglichen Bezugsproblemen mit dem Lieferanten des Pumpenkopfes erläutert werden. Sollte dieser Fall eintreten, wäre es möglich, vor dem Zusammenfügen von Patientenmodulen mit einem neuen Pumpenkopf das Steuermodul mit einer entsprechenden Adaption zu modifizieren und zu überprüfen.

Alle Komponenten des Steuermoduls bedürfen einer regelmäßigen Überprüfung und Kalibrierung. Die Einhaltung dieser Inspektionsintervalle entfällt mit diesem Konzept, da ausschließlich das Basismodul beim Anwender verbleibt, was jedoch im wesentlichen keine sicherheitsrelevanten Komponenten enthält.

Nach Rücksprache mit den entsprechenden medizinischen Kompetenzen könnte es möglich sein, auf dem Steuermodul einen Satz fester Notprogramme zu implementieren, die bei Ausfall des Basismoduls unter Zuhilfenahme externer Energiequellen (z.B. KFZ-Batterie) zum Einsatz kommen. Diese sind dann zwar nicht optimal, und auf die Aufzeichnung ausführlicher Betriebsdaten muss verzichtet werden, stellen aber eine bessere Alternative als den Totalausfall des Systems dar.

Im Katastropheneinsatz oder bei Massenunfällen kann es wünschenswert sein, mehrere Herzlungenmaschine-Systeme zum Einsatz zu bringen. Durch die Anzahl von Basismodulen vor Ort wäre man hierbei auf die Anzahl der Anwendungsfälle begrenzt. Durch die erfindungsgemäße Herzlungenmaschine ist es möglich, die Einstellungen des Systems und das Priming mit dem Basismodul durchzuführen, es danach vom Steuermodul zu trennen und danach Steuermodul und Patientenmodul mit einer externen Energiequelle weiter zu betreiben. Das stünde dann für die Behandlung eines weiteren Patienten zur Verfügung. Somit würde lediglich die Zahl der Steuermodul/Patientenmodul-Einheiten die Zahl der behandelten Patienten begrenzen.

### Bezugszeichenliste

- 1: Patientenmodul
- 2: Steuermodul
- 3: Basismodul
- 4: Reservoir
- 5: Pumpe
- 6: Oxygenator
- 7: Wärmetauscher
- 8: arterieller Filter
- 9: Antrieb
- 10: Handkurbel
- 11: Mess- und Steuerelektronik
- 12: Backup-Akku
- 13: Touchscreen
- 14: Computer
- 15: Hauptenergieversorgung

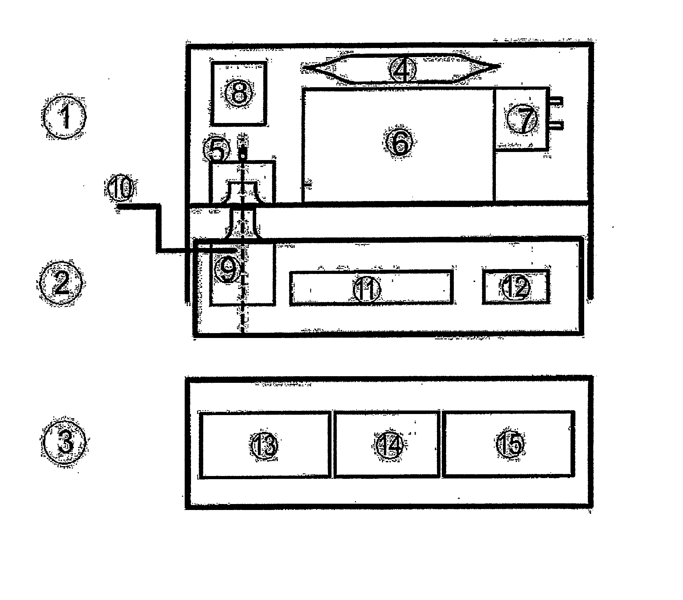

## Patentansprüche

1. Herzlungenmaschine, die von einer Person tragbar und unabhängig von externen Stromquellen einsetzbar ist und die zwei trennbar funktionell verbundene Module (1, 2) und eine Programmiereinheit (14) umfasst, worin
ein erstes Modul (1) die für die Funktion der Herzlungenmaschine erforderlichen blutführenden Bauteile aufweist, und
ein zweites Modul (2) einen Pumpenantrieb (9) und eine Meß- und Steuerelektronik (11) aufweist,
**dadurchgekennzeichnet** daß
die Programmiereinheit (14) in einem dritten Modul (3) vorgesehen ist, das mit dem zweiten Modul (2) trennbar funktionell verbunden ist, wobei das zweite Modul (2) Ventile für die Einstellung eines internen Bypasses aufweist.

2. Herzlungenmaschine nach Anspruch 1, die aus drei Modulen besteht.

3. Herzlungenmaschine nach Anspruch 1 oder 2, worin die Module (1, 2, 3) mittels Halteelementen lösbar aneinander fixierbar sind.

4. Herzlungenmaschine nach einem der vorstehenden Ansprüche, worin das zweite Modul (2) eine Übergangsenergieversorgung und Bedieneinheiten für den Notbetrieb aufweist.

5. Herzlungenmaschine nach einem der vorstehenden Ansprüche, worin das zweite Modul (2) ein Speichermedium zur Übertragung von Informationen von dem dritten Modul auf ein anderes drittes Modul aufweist.

6. Herzlungenmaschine nach einem der vorstehenden Ansprüche, worin das dritte Modul (3) eine Bedienoberfläche (13), einen Computer (14) und eine Hauptstromquelle (15) aufweist.

7. Herzlungenmaschine nach einem der vorstehenden Ansprüche, worin das erste und das zweite Modul (1, 2) zusammensteckbar und von einer Person tragbar sind.

8. Herzlungenmaschine nach Anspruch 7, worin das erste und das zweite Modul ohne das dritte Modul die Funktionen einer Herzlungenmaschine erfüllen können.

9. Herzlungenmaschine nach einem der vorstehenden Ansprüche, worin die Verbindung zwischen dem zweiten Modul und dem dritten Modul durch eine einfache elektrische Verbindung hergestellt wird, die sich beim Zusammensetzen der Module zwangsweise verbindet.

10. Herzlungenmaschine, die aus einem ersten Modul (1) und einem zweiten Modul (2) besteht, wobei das erste Modul (1) die für die Funktion der Herzlungenmaschine erforderlichen blutführenden Bauteile aufweist, und das zweite Modul (2) einen Pumpenantrieb (9), eine Meß- und Steuerelektronik (11) und eine Übergangsenergieversorgung aufweist
**dadurch gekennzeichnet daß**
das zweite Modul (2) Ventile für die Einstellung eines internen Bypasses und Bedieneinheiten für den Notbetrieb aufweist.

11. Steuermodul für eine Herzlungenmaschine, umfassend:
einen Pumpenantrieb (9), eine Meß- und Steuerelektronik (11), Ventile für die Einstellung eines internen Bypasses und ein Speichermedium zur Übertragung von Informationen von einem mit einer Programmiereinheit (14) versehenen Basismodul (3) auf ein anderes mit einer Programmiereinheit (14) versehenes Basismodul (3).

12. Verfahren zur Herstellung einer Herzlungenmaschine nach Anspruch 10, welches das Verbinden des ersten Moduls (1) mit dem zweiten Modul (2) umfaßt.

13. Verfahren nach Anspruch 12,
**dadurchgekennzeichnet** ,daß
das Verbinden werksseitig vorgenommen wird und daß beide Module nach dem Verbinden gemeinsam einer Funktionsprüfung und Kalibrierung unterzogen werden.

## Claims

1. A heart-lung machine which can be carried by one person and can be used independently of external power supplies and which comprises two separably and functionally connected modules (1, 2) and a programming unit (14), wherein
a first module (1) has the blood circulating elements required for the function of the heart-lung machine, and
a second module (2) has a pump drive (9) and measurement and control electronics (11),
**characterized in that**
the programming unit (14) is provided in a third module (3) separably and functionally connected to the second module (2), wherein the second module (2) has valves for adjusting an internal bypass.

2. A heart-lung machine in accordance with claim 1, consisting of three modules.

3. A heart-lung machine in accordance with claim 1 or claim 2, wherein the modules (1, 2, 3) are releasably fixable to one another by means of holding elements.

4. A heart-lung machine in accordance with any one of the preceding claims, wherein the second module (2) has a transition energy supply and operating units for emergency operation.

5. A heart-lung machine in accordance with any one of the preceding claims, wherein the second module (2) has a storage medium for transferring information from the third module to another third module.

6. A heart-lung machine in accordance with any one of the preceding claims, wherein the third module (3) has a user interface (13), a computer (14) and a main power supply (15).

7. A heart-lung machine in accordance with any one of the preceding claims, wherein the first and the second modules (1, 2) can be joined together and can be carried by one person.

8. A heart-lung machine in accordance with claim 7, wherein the first and the second modules can satisfy the functions of a heart-lung machine.

9. A heart-lung machine in accordance with any one of the preceding claims, wherein the connection between the second and the third module is established by a simple electrical connection which connects in a compulsory manner on the assembly of the modules.

10. A heart-lung machine consisting of a first module (1) and a second module (2), wherein
the first module (1) has the blood circulating elements required for the function of the heart-lung machine, and
the second module (2) has a pump drive (9), measurement and control electronics (11) and a transition emergency energy supply,
**characterized in that**
the second module (2) has valves for adjusting an internal bypass and operating units for emergency operation.

11. A control module for a heart-lung machine, comprising:
a pump drive (9), measurement and control electronics (11), valves for adjusting an internal bypass and a storage medium for transferring information from a base module (3) provided with a programming unit (14) to another base module (3) provided with a programming unit (14).

12. A method for manufacturing a heart-lung machine in accordance with claim 10, which comprises the connecting of the first module (1) to the second module (2).

13. A method in accordance with claim 12, **characterized in that** the connection is made ex works; and **in that** both modules are subjected to a function test and to calibration together after connection.

## Revendications

1. Coeur-poumon artificiel, susceptible d'être porté par une personne et d'être utilisé indépendamment de sources de courant externes, qui comprend deux modules (1, 2) reliés de façon fonctionnelle et séparables et une unité de programmation (14), dans laquelle
un premier module (1) comprend les composants de circulation sanguine nécessaire pour la fonction du coeur-poumon artificiel, et
un deuxième module (2) comprend un entraînement de pompe (9) et unité électronique de mesure et de commande (11),
**caractérisé en ce que**
l'unité de programmation (14) est prévue dans un troisième module (3) qui est relié de façon fonctionnelle et séparable au deuxième module (2), et le deuxième module (2) comprend des valves pour la réalisation d'un by-pass interne.

2. Coeur-poumon artificiel et selon la revendication 1, constitué de trois modules.

3. Coeur-poumon artificiel selon la revendication 1 ou 2, dans lequel les modules (1, 2, 3) sont capables d'être fixés de façon détachable les uns aux autres au moyen d'éléments de maintien.

4. Coeur-poumon artificiel selon l'une des revendications précédentes, dans lequel le deuxième module (2) comprend une alimentation énergétique de transition et des unités de service pour le fonctionnement de secours.

5. Coeur-poumon artificiel selon l'une des revendications précédentes, dans lesquels le deuxième module (2) comprend un support mémoire pour la transmission d'informations du troisième module à un autre troisième module.

6. Coeur-poumon artificiel selon l'une des revendications précédentes, dans lequel le troisième module (3) comporte une interface utilisateur (13), un calculateur (14) et une source de courant principale (15).

7. Coeur-poumon artificiel selon l'une des revendications précédentes, dans lequel le premier et le deuxième module (1, 2) peuvent être enfichés conjointement et portable par une personne.

8. Coeur-poumon artificiel selon la revendication 7, dans lequel le premier et le deuxième module peuvent remplir les fonctions d'un coeur-poumon artificiel sans le troisième module.

9. Coeur-poumon artificiel selon l'une des revendications précédentes, dans lequel la liaison entre le deuxième module et le troisième module est établie par une simple liaison électrique qui s'établit obligatoirement lors de l'assemblage des modules.

10. Coeur-poumon artificiel, constitué d'un premier module (1) et d'un deuxième module (2), dans lequel
le deuxième module (1) comprend les composants de circulation sanguine nécessaire pour la fonction du coeur-poumon artificiel, et
le deuxième module comprend un entraînement de pompe (9), une unité électronique de mesure et de commande (11), et une alimentation énergétique de transition,
**caractérisé en ce que**
le deuxième module (2) comprend des valves pour la réalisation d'un by-pass interne et des unités de service pour le fonctionnement de secours.

11. Module de commande pour un coeur-poumon artificiel, comprenant :
un entraînement de pompe (9), une unité électronique de mesure et de commande (11), des valves pour la réalisation d'un by-pass interne, et un support mémoire pour la transmission d'informations depuis un module de base (3) doté d'une unité de programmation (14) vers un autre module de base (3) doté d'une unité de programmation (14).

12. Procédé pour réaliser un coeur-poumon artificiel selon la revendication 10, qui comprend la liaison du premier module (1) au deuxième module (2).

13. Procédé selon la revendication 12, **caractérisé en ce que** la liaison est exécutée en usine, et **en ce qu'**après la liaison, les deux modules sont soumis conjointement à un contrôle de fonctionnement et à un calibrage.
